# EUROPÄISCHE PATENTSCHRIFT

(11) **EP 2 340 854 B1**
(45) Veröffentlichungstag und Bekanntmachung des Hinweises auf die Patenterteilung: **11.07.2012**
(21) Anmeldenummer: 09306232.1
(22) Anmeldetag: 15.12.2009
(51) Int. Cl.: A61L 9/12, A61L 9/14, F24F 3/12, F24F 3/16

(54) **Vorrichtung und Verfahren zum Entfernen von Schadstoffen**
Device and method for removing contaminants
Dispositif et procédé destinés à enlever des substances toxiques

(43) Veröffentlichungstag der Anmeldung: 06.07.2011
(73) Patentinhaber: Air & D- Sarl, 67190 Rosheim (FR)
(72) Erfinder: Wuest, Robert, 67560, ROSHEIM (FR)
(74) Vertreter: Nuss, Laurent

(56) Entgegenhaltungen:
- EP-A1- 0 608 176
- EP-A1- 1 882 481
- WO-A1-2009/083157
- CA-A1- 2 378 043

## Beschreibung

Die Erfindung betrifft eine Vorrichtung und ein Verfahren zum Entfernen bzw. Vermindern von schädlichen bzw. übel riechenden Substanzen aus der Luft von Gebäuden.

In dem deutschen Gebrauchsmuster DE-U 20 2007 017 927 sowie in der Patentanmeldung WO 2009/083157 ist eine Vorrichtung zum Behandeln der Umgebungsluft mit aktiven Agenzien beschrieben. Diese Vorrichtung umfasst einen Kasten mit Platten aus einem gelförmigen Trägermaterial, an dem flüchtige aktive Agenzien adsorbiert sind. Zwangsgeförderte Luft streicht an den Platten vorbei und setzt die aktiven Agenzien frei. Ein Kompressor saugt die mit den aktiven Agenzien beladene Luft an, komprimiert sie und presst sie durch Rohre bzw. Schläuche aus dem Kasten aus, wodurch sie an verschiedene Stellen eines Raumes befördert werden kann.

In der Patentanmeldung EP-A 1 882 481 ist eine Vorrichtung und ein Verfahren zum Desodorieren von geschlossenen Räumen oder großflächigen Anlagen beschrieben, bei dem verunreinigte Luft mit flüchtigen, aktiven Agenzien behandelt wird, die mittels Druckluft mit Hilfe des Venturi- Prinzips durch Düsen an die Umgebungsluft versprüht werden.

Die Patentanmeldung CA-2 378 043 offenbart eine Vorrichtung und ein Verfahren zur Luftbehandlung in geschlossenen Räumen, wobei verunreinigte Luft mit eingespritzten flüssigen Agenzien innerhalb eines Gehäuses behandelt wird.

Es hat sich nun gezeigt, dass in beiden Fällen die aktiven Agenzien dabei nicht gleichmäßig in dem Raum verteilt werden, so dass an vielen Stellen des Raumes keine Behandlung der verunreinigten Luft erfolgt. Der Erfindung lag daher die Aufgabe zu Grunde, diesen Missstand zu beheben. Diese Aufgabe wird durch die erfindungsgemäße Vorrichtung und das erfindungsgemäße Verfahren gelöst.

Die Erfindung wird in den Hauptansprüchen definiert und charakterisiert, während die abhängigen Ansprüche weitere Merkmale der Erfindung beschreiben.

Gegenstand der Erfindung ist demzufolge eine Vorrichtung zum Entfernen bzw. Vermindern von schädlichen bzw. übel riechenden Substanzen aus der Luft von Gebäuden in Form eines Gehäuses, welches enthält:
- ein Einlassrohr oder einen Einlassschlauch oder mehrere Rohre bzw. Schläuche, durch welche aktive Agenzien eingeführt werden können,
- eine Eintrittsstelle in Form einer oder mehrerer Öffnungen, durch welche Luft aus dem umgebenden Raum eintreten kann,
- eine Einrichtung zum Ansaugen von Umgebungsluft durch die Eintrittsstelle in das Gehäuse und zum Befördern dieser Umgebungsluft zusammen mit, der mit den aktiven Agenzien beladene Luft aus dem Gehäuse heraus, und,
- eine Austrittsöffnung, durch welche die mit den aktiven Agenzien beladene Luft in den Raum austreten kann.

Diese und andere Merkmale der Erfindung ergeben sich aus der folgenden Beschreibung bevorzugter Ausführungsformen der Erfindung, die nicht einschränkende Beispiele darstellen und in der auf die folgenden Zeichnungen Bezug genommen wird:

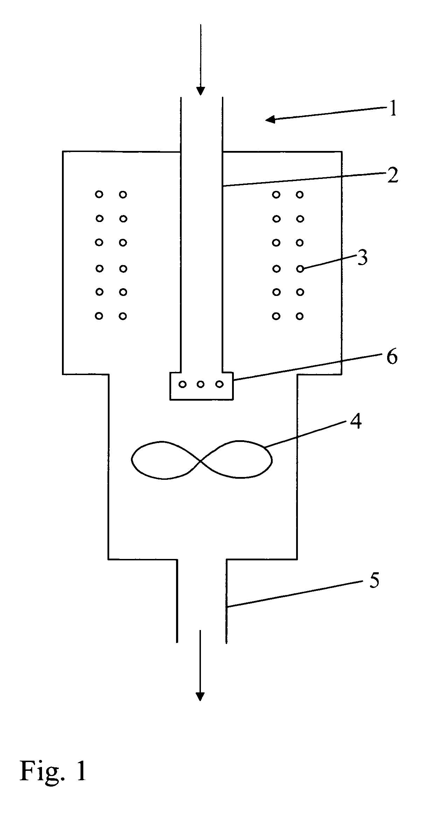

Fig. 1 ist ein Längsschnitt einer ersten erfindungsgemäßen Vorrichtung,

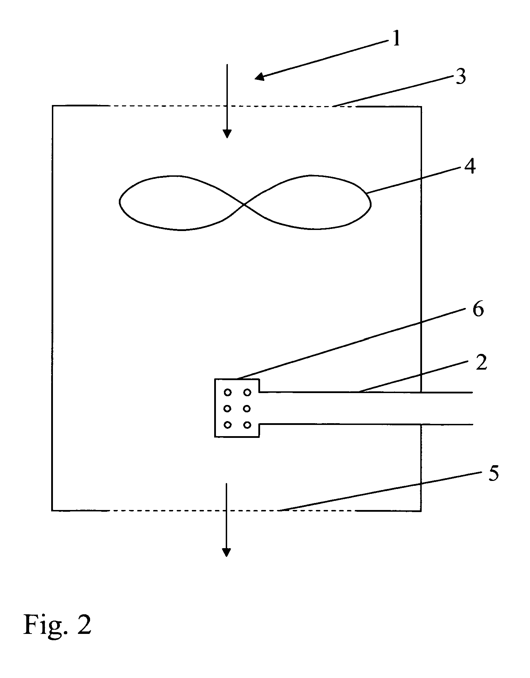

Fig. 2 ist ein Längsschnitt einer zweiten erfindungsgemäßen Vorrichtung.

Die Abbildungen zeigen schematisch zwei Ausführungsformen der erfindungsgemäßen Vorrichtung. Dabei ist jeweils mit 2 das Einlassrohr bzw. der Einlassschlauch bezeichnet, mit 3 die Eintrittsstelle für die Umgebungsluft, mit 4 die Einrichtung zum Ansaugen der Umgebungsluft und Befördern der Luft aus dem Gehäuse 1, mit 5 die Austrittsöffnung und mit 6 den Verteilerkopf auf das Einlassrohr bzw. den Einlassschlauch mit Öffnungen.

Gemäß den Zeichnungen enthält die erfindungsgemäße Vorrichtung ein Gehäuse 1, welches enthält:
- ein Einlassrohr oder einen Einlassschlauch 2 oder mehrere Rohre bzw. Schläuche, durch welche mit aktiven Agenzien beladene Luft eingeführt werden können,
- eine Eintrittsstelle 3 in Form einer oder mehrerer Öffnungen, durch welche Luft aus dem umgebenden Raum eintreten kann,
- eine Einrichtung 4 zum Ansaugen von Umgebungsluft durch die Eintrittsstelle 3 in das Gehäuse 1 und zum Befördern dieser Luft zusammen mit, der mit den aktiven Agenzien beladenen Luft aus dem Gehäuse 1, und
- eine Austrittsöffnung 5, durch welche die beladene Luft in den Raum austreten kann.

Das Gehäuse 1 besteht vorzugsweise aus Blech, Edelstahl oder PVC. Es kann einen Rauminhalt je nach Bedarf von z.B. 10 bis 1000 Liter umfassen. Es enthält ein Einlassrohr bzw. einen Einlassschlauch 2, oder auch mehrere Rohre bzw. Schläuche, durch die die mit aktiven Agenzien beladene Luft eingeführt werden kann. Diese stammen vorzugsweise aus den in den oben genannten Schriften beschriebenen Vorrichtungen, in denen sie mittels Luft freigesetzt und ausgepresst wurden. Die aktiven Agenzien treten also mit Luft vermischt in das Gehäuse 1 ein. Das Einlassrohr bzw. der Einlassschlauch 2 ist vorzugsweise an seinem vorderen Ende mit einem oder mehreren ringförmigen Verteilerköpfen 6 versehen, welche an ihrem Umfang eine oder mehrere, insbesondere 2 bis 10 Öffnungen in Form von Löchern oder Düsen enthalten, durch welche die mit aktiven Agenzien beladene Luft in dem Gehäuse gleichmäßig verteilt werden können.

Weiter enthält das Gehäuse 1 eine Eintrittsstelle 3, durch welche Luft aus dem umgebenden Raum eintreten kann. Dabei kann es sich entweder um eine einzige, größere Öffnung handeln oder es können in die Wand des Gehäuses 1 eine Vielzahl von Öffnungen, z.B. in Form von Löchern oder als Gitter eingelassen sein.

Ferner enthält das Gehäuse 1 eine Einrichtung 4 zum Ansaugen von Umgebungsluft durch die Eintrittsstelle 3 und zum Befördern dieser Luft zusammen mit der mit den aktiven Agenzien beladenen Luft aus dem Gehäuse 1. Diese Einrichtung 4 muss eine Pumpfunktion ausüben. Es kann sich dabei vorzugsweise um einen kräftigen Ventilator, eine Turbine oder auch um eine Flügelzellenpumpe oder eine Flügelradpumpe handeln. Durch diese Einrichtung 4 wird die eingesaugte Luft mit der durch das Einlassrohr bzw. den Einlassschlauch 2 eingeführten, mit aktiven Agenzien beladenen Luft vermischt, und die verdünnte, beladene Luft wird durch eine Austrittsöffnung 5 aus dem Gehäuse 1 hinaus befördert. Von der Austrittsöffnung 5 tritt die beladene Luft entweder direkt in den Raum aus oder sie kann auch von einem Austrittsrohr in mehrere Rohre verzweigt an verschiedene Stellen des Raums befördert werden.

Bei kleineren, z.B. kastenförmigen Gehäusen 1 mit einem Volumen von vorzugsweise 20 bis 200 Liter befindet sich das Einlassrohr bzw. der Einlassschlauch 2 in einer Eintrittsstelle 3 des Gehäuses 1 hinter dem Ventilator und dort befinden sich auch Öffnungen bzw. Gitter in der Gehäusewand. Der Ventilator befördert dann das Luftgemisch mit den aktiven Agenzien in einen Austrittsbereich des Gehäuses mit der Austrittsöffnung 5.

Bei größeren, z.B. rohrförmigen Gehäusen 1 mit einem Volumen von vorzugsweise 200 bis 800 Liter kann sich an seinem hinteren Ende eine große Öffnung, ein Gitter oder ein Lochblech befinden, durch die Umgebungsluft eingesaugt wird. In diesem Fall kann das Einlassrohr bzw. der Einlassschlauch 2 auch vor dem Ventilator angebracht sein, so dass sich im Austrittsbereich die Luft vermischt.

Vorzugsweise ist die erfindungsgemäße Vorrichtung im Decken- bzw. im Dachbereich des Gebäudes angebracht, z.B. aufgehängt, so dass die Austrittsöffnung 5, die z.B. die Form eines kurzen Rohrs haben kann, nach unten gerichtet ist, wobei dann die beladene Luft nach unten in das Gebäude ausströmt. Dabei kann die Austrittsöffnung 5 ringförmige, nach außen gerichtete Lamellen aufweisen, durch welche die beladene Luft seitlich, insbesondere waagrecht ausströmen kann. Dies ist dann zweckmäßig, wenn die beladenen Luft nicht direkt auf darunter sich befindende Menschen oder Tiere auftreffen soll.

Bei einer weiteren Ausführungsform (nicht dargestellt) befindet sich die Eintrittsstelle 3 für die Luft an der Unterseite des Gehäuses 1 und eine oder mehrere Austrittsöffnungen 5 an der Seite des Gehäuses 1, so dass die mit den aktiven Agenzien beladene Luft seitlich in den Raum hinein austreten kann.

Grundsätzlich kann die erfindungsgemäße Vorrichtung auch im unteren Bereich des Raums angebracht sein.

Ein weiterer Gegenstand der Erfindung ist ein Verfahren zum Entfernen bzw. Vermindern von schädlichen bzw. übel riechenden Substanzen aus der Luft von Gebäuden, bei dem man
- mit aktiven Agenzien beladene Luft in ein Gehäuse, insbesondere ein erfindungsgemäßes Gehäuse 1, einführt,
- in das Gehäuse zusätzlich Umgebungsluft ansaugt, und,
- die angesaugte Luft zusammen mit der mit aktiven Agenzien beladenen Luft aus dem Gehäuse in das Gebäude ausbläst.

Vorzugsweise stammt die mit aktiven Agenzien beladene Luft aus einem Kasten nach DE-U 20 2007 017 927 bzw. WO 2009/083157, wobei man
- Umgebungsluft in einen Kasten hineinbefördert, beispielsweise mittels eines Ventilators ansaugt,
- die Luft an gelförmigen Trägerelementen, an denen flüchtige aktive Agenzien adsorbiert sind, vorbeiführt und die aktiven Agenzien dabei freisetzt, und die Luft damit anreichert,
- mittels eines Kompressors die beladene Luft ansaugt, komprimiert, und
- die komprimierte Luft durch ein oder mehrere Rohre oder Schläuche in ein Gehäuse, insbesondere ein erfindungsgemäßes Gehäuse 1, auspresst.

Die mit den aktiven Agenzien beladene Luft kann auch aus einem Kasten nach EP-A 1 882 481 stammen, wobei man
- Druckluft in den Gasraum eines Behälters mit flüchtigen, aktiven Agenzien einleitet,
- die Druckluft durch Düsen auspresst, wobei nach dem Venturi-Effekt durch Unterdruck aktive Agenzien aus dem Behälter herausgesaugt werden,
- wonach die durch die Düsen zusammen mit Luft ausgepressten aktiven Agenzien nach dem Venturi-Prinzips zerstäubt werden, und
- die mit den aktiven Agenzien beladene Luft durch ein Rohr oder einen Schlauch in ein Gehäuse, insbesondere ein erfindungsgemäßes Gehäuse 1, einführt.

Wie in den zitierten Schriften beschrieben, kann die in den Kästen mit aktiven Agenzien beladene Luft aus mehreren Schläuchen oder Rohren ausgepresst werden. Aus diesen wird dann das Gehäuse, insbesondere das erfindungsgemäße Gehäuse 1, gespeist. Das heißt, dass aus einem einzigen Kasten eine Vielzahl von Gehäusen mit der beladenen Luft beschickt werden kann.

Bei der bevorzugten Ausführungsform der Erfindung ist das Gehäuse, insbesondere das erfindungsgemäße Gehäuse 1, im Decken- bzw. im Dachbereich des Gebäudes aufgehängt, und die beladene Luft kann durch die Austrittsöffnung 5 direkt nach unten ausströmen oder durch ringförmige Lamellenöffnungen seitlich aus dem besagten Gehäuse austreten.

Die mit den aktiven Agenzien beladene Luft durchströmt dann den Innenraum des Gebäudes und entfaltet ihre desodorierende Wirkung. Ein Teil der Luft wird wieder in das besagten Gehäuse eingesaugt, vermischt sich dort mit der aus dem Einlassrohr ausgepressten, frisch mit aktiven Agenzien beladenen Luft und wird erneut in den Raum hinein befördert. Durch diesen Kreislauf wird bewirkt, dass die mit den aktiven Agenzien beladene Luft den gesamten Innenraum des Gebäudes bestreichen kann, insbesondere, wenn mehrere, beispielsweise 2 bis 20 Gehäuse in dem Raum angebracht sind. Die mit den Schadstoffen kontaminierte Luft in dem Gebäude kommt mehrere Male mit aktiven Agenzien in Berührung, so dass die Schadstoffe vollständig oder wenigstens weitgehend entfernt werden.

Die Erfindung eignet sich zum Entfernen bzw. Vermindern von schädlichen bzw. übel riechenden Substanzen in der Luft von geschlossenen oder halboffenen Gebäuden, z. B. von Agrarindustrieanlagen, Kompostieranlagen, Großställen, Großküchen, Parkhäusern, Supermärkten, Verkaufshallen, Pumpstationen und Entwässerungsräumen.

Schädliche bzw. übelriechende Substanzen, die erfindungsgemäß aus den Räumen entfernt bzw. vermindert werden sollen, sind z.B. Ammoniak, Amine, Schwefelwasserstoff, Mercaptane, Kohlenwasserstoffe und Lösungsmitteldämpfe.

Aktive Agenzien sind z.B. Aldehyde, Ketone, Alkohole, Terpene oder Ester sowie deren Mischungen, ferner Desodorantien, Insektizide, Biozide, die in dem Kasten nach DE-U 20 2007 017 927 an gelförmigen Trägermaterialien adsorbiert sind, oder sich in dem Behälter nach EP-A 1 882 481 befinden, und daraus freigesetzt werden. Geeignete aktive Agenzien und Trägermaterialien sind in EP-A 1 334 735 ausführlich beschrieben.

Es wird angenommen, dass die aktiven Agenzien mit den schädlichen bzw. übel riechenden Substanzen chemisch reagieren und sie abbauen oder modifizieren und dadurch entfernen.

## Patentansprüche

1. Vorrichtung zum Entfernen bzw. Vermindern von schädlichen bzw. übel riechenden Substanzen aus der Luft von Gebäuden in Form eines Gehäuses (1), welches enthält:
- ein Einlassrohr oder einen Einlassschlauch (2) oder mehrere Rohre bzw. Schläuche, durch welche mit aktiven Agenzien beladene Luft eingeführt werden können,
- eine Eintrittsstelle (3) in Form einer oder mehrerer Öffnungen, durch welche Luft aus dem umgebenden Raum eintreten kann,
- eine Einrichtung (4) zum Ansaugen von Umgebungsluft durch die Eintrittsstelle (3) in das Gehäuse (1) und zum Befördern dieser Umgebungsluft zusammen mit der mit den aktiven Agenzien beladenen Luft aus dem Gehäuse (1), und,
- eine Austrittsöffnung (5), durch welche die beladene Luft in den Raum austreten kann.

2. Vorrichtung nach Anspruch 1, **dadurch gekennzeichnet, dass** das Einlassrohr bzw. der Einlassschlauch (2) mit einem Verteilerkopf (6) oder mehreren Verteilerköpfen mit einer oder mehreren Öffnungen versehen ist, durch welche die mit aktiven Agenzien beladene Luft in dem Gehäuse (1) gleichmäßig verteilt werden können.

3. Vorrichtung nach Anspruch 1, **dadurch gekennzeichnet, dass** sie im Decken- bzw. Dachbereich des Gebäudes angebracht ist, und dass die durch die Austrittsöffnung (5) austretende Luft nach unten in das Gebäude hinein geführt wird.

4. Vorrichtung nach Anspruch 1, **dadurch gekennzeichnet, dass** die Austrittsöffnung (5) ringförmige Lamellenöffnungen aufweist, durch welche die Luft seitlich austreten kann.

5. Vorrichtung nach Anspruch 1, **dadurch gekennzeichnet, dass** die Einrichtung (4) zum Ansaugen von Luft ein Ventilator ist.

6. Verfahren zum Entfernen bzw. Vermindern von schädlichen bzw. übel riechenden Substanzen aus der Luft von Gebäuden, **dadurch gekennzeichnet, dass** man
- mit aktiven Agenzien beladene Luft durch ein Einlassrohr oder einen Einlassschlauch (2) oder mehrere Rohre bzw. Schläuche einführt in ein Gehäuse (1) einführt,
- in das Gehäuse (1) zusätzlich Umgebungsluft durch eine weitere Eintrittsstelle (3) in Form einer oder mehrerer Öffnungen ansaugt, und,
- die zusätzlich angesaugte Luft wird zusammen mit der mit aktiven Agenzien beladenen Luft vermischt und dann durch eine Austrittöffnung (5) aus dem Gehäuse (1) in das Gebäude ausgeblasen.

7. Verfahren nach Anspruch 6, bei dem man
- Umgebungsluft in einen Kasten hineinbefördert,
- die Luft an Trägerelementen für flüchtige aktive Agenzien vorbeiführt, wobei die aktiven Agenzien freigesetzt werden und die Luft damit angereichert wird,
- die mit den aktiven Agenzien beladene Luft komprimiert und,
- die komprimierte, mit den aktiven Agenzien beladene Luft aus dem Kasten auspresst, **dadurch gekennzeichnet, dass** man
- die ausgepresste, mit den aktiven Agenzien beladene Luft in ein Gehäuse (1) einführt,
- in das Gehäuse (1) zusätzlich Umgebungsluft ansaugt, und
- die angesaugte Luft zusammen mit der mit den aktiven Agenzien beladenen Luft aus dem Gehäuse (1) in das Gebäude ausbläst.

8. Verfahren nach Anspruch 6, bei dem man
- Druckluft in den Gasraum eines Behälters mit flüchtigen, aktiven Agenzien einleitet,
- die Druckluft durch Düsen auspresst, wobei nach dem Venturi-Effekt durch Unterdruck aktive Agenzien aus dem Behälter herausgesaugt werden,
- wonach die durch die Düsen zusammen mit Luft ausgepressten aktiven Agenzien nach dem Venturi-Prinzip zerstäubt werden,
**dadurch gekennzeichnet, dass** man
- die mit den aktiven Agenzien beladene Luft durch ein Rohr oder einen Schlauch (2) in ein Gehäuse (1) einführt,
- in das Gehäuse (1) zusätzlich Umgebungsluft ansaugt, und,
- die angesaugte Luft zusammen mit der mit den aktiven Agenzien beladenen Luft aus dem Gehäuse (1) in das Gebäude ausbläst.

9. Verfahren nach Anspruch 6, **dadurch gekennzeichnet, dass** die aus dem Gehäuse (1) ausgeblasene Luft sich in dem Gebäude gleichmäßig verteilt, sich dort mit der Umgebungsluft vermischt und diese im Kreis wiederum in das Gehäuse (1) eingesaugt wird.

## Claims

1. Device for removing or reducing harmful or malodorous substances from the air of buildings in the form of a housing (1) which contains:
- an inlet tube or an inlet hose (2) or a plurality of tubes or hoses through which air charged with active agents can be introduced,
- an inlet point (3) in the form of one or more openings through which air from the surrounding room can enter,
- a device (4) for suctioning the surrounding air through the inlet point (3) into the housing (1) and for conveying this surrounding air together with the air charged with active agents out of the housing (1) and
- an outlet opening (5) through which the charged air can escape into the room.

2. Device according to claim 1, **characterised in that** the inlet tube or the inlet hose (2) is provided with a distributor head (6) or a plurality of distributor heads comprising one or more openings, through which the air charged with active agents can be distributed evenly in the housing (1).

3. Device according to claim 1, **characterised in that** it is attached to the ceiling or roof area of the building and **in that** the air exiting through the outlet opening (5) is guided downwards into the building.

4. Device according to claim 1, **characterised in that** the outlet opening (5) comprises annular lamella openings through which the air can exit laterally.

5. Device according to claim 1, **characterised in that** the device (4) for suctioning in air is a ventilator.

6. Method for removing or reducing harmful or malodorous substances from the air of buildings, **characterised in that**
- air charged with active agents is introduced through an inlet tube or an inlet hose (2) or a plurality of tubes or hoses into a housing (1),
- surrounding air is also suctioned into the housing (1) through an additional inlet point (3) in the form of one or more openings and,
- the additional suctioned air is mixed together with the air charged with active agents and is then blown out of the housing (1) through an outlet opening (5) into the building.

7. Method according to claim 6, in which
- surrounding air is conveyed into a box,
- the air is moved past carrier elements for volatile active agents, where the active agents are released and the air is enriched with the latter,
- the air charged with the active agents is compressed and
- the compressed air charged with the active agents is pressed out of the box, **characterised in that**
- the pressed our air charged with the active agents is introduced into a housing (1),
- additional surrounding air is suctioned into the housing (1) and
- the suctioned air together with the air charged with active agents is blown out of the housing (1) into the building.

8. Method according to claim 6 in which
- compressed air is introduced into the gas chamber of a container with volatile active agents,
- the compressed air is pressed out through nozzles, wherein according to Venturi effect by vacuum active agents are suctioned out of the container,
- after which the active agents pressed out through the nozzles together with air are atomized according to the Venturi principle,
**characterised in that**
- the air charged with active agents is introduced through a tube or a hose (2) into a housing (1),
- additional surrounding air is suctioned into the housing (1) and
- the suctioned air together with the air charged with active agents is blown out of the housing (1) into the building.

9. Method according to claim 6, **characterised in that** the air blown out of the housing (1) is distributed evenly in the building, is mixed there with the surrounding air and the latter is suctioned in a circuit back into the housing (1).

## Revendications

1. Dispositif pour éliminer ou, respectivement, pour atténuer les substances respectivement nocives ou malodorantes renfermées par l'air présent dans des bâtiments, revêtant la forme d'un boîtier (1) comprenant :
- une tubulure d'admission ou un tuyau souple d'arrivée (2), voire plusieurs tubulures ou tuyaux souples permettant l'introduction d'air chargé en composants actifs,
- une zone d'entrée (3), revêtant la forme d'un ou de plusieurs orifice(s) permettant la pénétration d'air en provenance de l'espace environnant,
- un système (4) pour aspirer de l'air ambiant dans le boîtier (1), par l'intermédiaire de la zone d'entrée (3), et pour charrier cet air ambiant hors dudit boîtier (1), conjointement à l'air chargé en composants actifs, et
- un orifice de sortie (5), par lequel ledit air chargé peut être évacué pour pénétrer dans l'espace.

2. Dispositif selon la revendication 1, **caractérisé par le fait que** la tubulure d'admission, ou le tuyau souple d'arrivée (2), est respectivement doté(e) d'une tête répartitrice ou de distribution (6) ou de plusieurs têtes répartitrices ou de distribution présentant un ou plusieurs orifice(s) par le(s)quel(s) l'air, chargé en composants actifs, peut être uniformément réparti dans le boîtier (1).

3. Dispositif selon la revendication 1, **caractérisé par le fait qu'**il est installé dans la région du plafond ou, respectivement, du toit du bâtiment ; et **par le fait que** l'air, évacué par l'orifice de sortie (5), est introduit vers le bas dans ledit bâtiment.

4. Dispositif selon la revendication 1, **caractérisé par le fait que** l'orifice de sortie (5) comporte des ouvertures à lamelles annulaires, par lesquelles l'air peut sortir latéralement.

5. Dispositif selon la revendication 1, **caractérisé par le fait que** le système (4) d'aspiration d'air est un ventilateur.

6. Procédé conçu pour éliminer ou, respectivement, pour atténuer les substances respectivement nocives ou malodorantes renfermées par l'air présent dans des bâtiments, **caractérisé par le fait que**
- de l'air, chargé en composants actifs, est introduit dans un boîtier (1) en empruntant une tubulure d'admission ou un tuyau souple d'arrivée (2), voire plusieurs tubulures ou tuyaux souples,
- de l'air ambiant est additionnellement aspiré dans ledit boîtier (1), par l'intermédiaire d'une autre zone d'entrée (3) revêtant la forme d'un ou de plusieurs orifice(s), et
- l'air additionnellement aspiré est mélangé à l'air chargé en composants actifs, puis expulsé dudit boîtier (1) en empruntant un orifice de sortie (5), pour pénétrer dans le bâtiment.

7. Procédé selon la revendication 6, dans lequel
- de l'air ambiant est introduit dans un caisson,
- l'air est guidé en regard d'éléments de support dévolus à des composants actifs volatils, lesdits composants actifs étant alors libérés, et l'air étant enrichi par ces derniers,
- l'air renfermant la charge en composants actifs est comprimé, et
- l'air comprimé, renfermant la charge en composants actifs, est exprimé dudit caisson, **caractérisé par le fait que**
- l'air exprimé, renfermant la charge en composants actifs, est introduit dans un boîtier (1),
- de l'air ambiant est additionnellement aspiré dans ledit boîtier (1), et
- l'air aspiré est expulsé dudit boîtier (1), conjointement à l'air renfermant la charge en composants actifs, pour pénétrer dans le bâtiment.

8. Procédé selon la revendication 6, dans lequel
- de l'air comprimé est admis dans la chambre à gaz d'un récipient contenant des composants actifs volatils,
- ledit air comprimé est exprimé par des buses, des composants actifs étant alors aspirés hors dudit récipient par effet Venturi, sous l'action d'une dépression,
- lesdits composants actifs, exprimés par les buses conjointement à de l'air, sont ensuite atomisés par effet Venturi,
**caractérisé par le fait que**
- l'air, renfermant la charge en composants actifs, est introduit dans un boîtier (1) par l'intermédiaire d'une tubulure ou d'un tuyau souple (2),
- de l'air ambiant est additionnellement aspiré dans ledit boîtier (1), et
- l'air aspiré est expulsé dudit boîtier (1), conjointement à l'air renfermant la charge en composants actifs, pour pénétrer dans le bâtiment.

9. Procédé selon la revendication 6, **caractérisé par le fait que** l'air expulsé du boîtier (1) se répartit uniformément dans le bâtiment, dans lequel il se mélange à l'air ambiant, puis ce dernier est de nouveau aspiré dans ledit boîtier (1), en circuit fermé.
